# EUROPEAN PATENT APPLICATION

(11) **EP 1 109 019 A1**
(43) Date of publication of application: **20.06.2001**
(21) Application number: 99830776.3
(22) Date of filing: 15.12.1999
(51) Int. Cl.: G01N 33/50, G01N 33/569, C12Q 1/70, C12N 9/50

(54) **Method to evaluate the sensitivity of HIV variants to drugs able to inhibit the HIV protease**

(71) Applicant: BioStrands S.r.l., 34012 Trieste (IT)
(72) Inventor: Menzo, Stefano, Universita' Di Ancona, 60020 Ancona (IT); Clementi, Massimo, 60035 Iesi (AN) (IT)
(74) Representative: Capasso, Olga

(57) **Abstract**

A method to evaluate the sensitivity of an HIV variant from a biological sample to at least one drug able to inhibit the HIV protease is disclosed. The method comprises the steps of: a) amplifying the nucleic acid encoding the HIV protease from the biological sample, to obtain an amplified HIV protease coding sequence; b) cloning the amplified HIV protease coding sequence into a unique restriction site of a modified HIV molecular clone, wherein said modified HIV molecular clone is modified at least by deleting the protease coding sequence and by replacing it with the unique restriction site, to obtain a recombinant HIV molecular clone comprising and expressing the HIV protease coding sequence; c) introducing the recombinant HIV molecular clone comprising and expressing the HIV protease coding sequence into cultured cells, to obtain recombinant HIV transfected cells; d) measuring the level of HIV p24 antigen into the cell supernatant of the recombinant HIV transfected cells both in the presence and in the absence of the drug able to inhibit the HIV protease. The method is suitable for any HIV variant, both HIV-1 or HIV-2 variants. Diagnostic kits are also disclosed.

## Description

### Technical field

The invention relates to a method to evaluate the sensitivity of a HIV variant from a biological sample to at least one drug able to inhibit the HIV protease.

### Background art

Considerable efforts are currently being directed toward the development of anti-HIV-1 chemotherapeutic agents. Two classes of compounds presently undergoing pre-clinical and clinical evaluations, the nucleoside and non-nucleoside inhibitors of viral reverse transcriptase (RT), demonstrated to have a high antiviral activity, both in vitro and in vivo. However, RT inhibitors showed only a moderate and transient efficacy when administered in a monotherapy regimen (1,2), due to the emergence of resistant viral variants (3,4). The introduction of potent inhibitors of HIV-1 protease (5,6) in combined therapeutic regimens (7,8) resulted in a marked improvement of clinical response.

The combined regimens aim at reducing or, ideally, suppressing viral replication in infected subjects for years. However, a residual viral activity often persists during therapy, albeit at minimal levels. In these circumstances, the HIV-1 replication, in the presence of the strong selective pressure of antiviral compounds, represents the ideal condition for the emergence (and rapid selection) of drug-resistant viral variants with a low susceptibility to all classes of drugs (9,10). In most cases, viral variants resistant to one protease inhibitor showed cross-resistance to other PIs currently available (11,12). Therefore the combination therapy for HIV-1 infection requires the monitoring of viral load as well as testing viral load sensitivity.

Indeed, the routine use of reliable, real-time methods to test the sensitivity of replicative viral strains could drive a more effective therapeutic intervention in HIV-1 infection.

Unlike RT, HIV-1 protease displays a substantial genetic polymorphism and sensitivity to PIs cannot be reliably deduced only from the viral nucleotide sequence.

In the last few years, several assays for the analysis of the recombinant HIV-1 phenotype in the presence of PI have been proposed to evaluate the selection of viral drug-resistant variants in treated subjects (13-16). These assays include viral infection of permissive cells and the evaluation of the IC₅₀ (50% inhibitory concentration) for each inhibitory compound after transfection of molecular recombinants and recovery of chimeric viruses, thus requiring high safety laboratories (P3 level) and high costs. Moreover the assays tend to be unpractical, time-consuming, and expensive; all this, together with the need of culturing replicative viral clones in phenotypic assays, strongly hampers their widespread laboratory use.

PCT Application W097/27480 discloses a method of managing the chemotherapy of HIV positive patients comprising the steps of: 1) transfecting an HIV sensitive cell line with a sequence from the HIV *pol* gene amplified from clinical samples and with an HIV construct which is deleted of the above sequence; 2) culturing the transfected cells to obtain chimeric recombinant viruses; 3) assessing the phenotype sensitivity of the chimeric viruses to at least three inhibitors of the enzyme coded by HIV *pol* gene; 4) comparing with HIV wild type virus. As mentioned above the method is long, expensive, and must be performed in high safety laboratories.

### Disclosure of the invention

The authors of the present invention set up a new recombinant assay to test the HIV-1 phenotype to protease inhibitors (PIs), producing non-replicative viruses. Thus the assay could be performed in standard laboratories by trained personnel. Moreover, the assay results proves t to be much faster than the ones of the prior art, directly measuring the levels of HIV p24 antigen shortly after transfection and not requiring the production of infectious particles. The p24 antigen is a proteolytic product of the viral protease, and its secretion in the presence of adequate concentrations of protease inhibitors is indicative of a viral variant harbouring a drug resistant protease gene. On the contrary, the absence of p24 reduction under the above mentioned conditions reveals a drug sensitive protease gene. The assay is based on the use of modified HIV-1 molecular clones, deleted at the endogeneous protease coding sequence, preferably also at the env (coding sequence, more preferably also at the reverse transcriptase coding sequence, all capable of expressing viral exogenous protease sequences. The assay is able to evaluate the HIV-1 phenotype with respect to different degrees of resistance to PIs directly after transfection, within 5 days from blood sampling. A percent Sensitivity Index (SI%), i.e. the ratio between the results obtained from each clonal sequence and those from a PI-sensitive reference strain, is calculated both in the presence and in the absence of the tested drug. By virtue of modifications introduced in the viral backbone (allowing the efficient cloning and expression of exogenous viral protease sequences and the production of non-infectious viral particles), the assay proves to be fast, safe, and sufficiently versatile to achieve the rational management of different anti-HIV-1 treatment regimens, as well as contributing to the understanding of HIV-1 protease resistance.

The method of the invention is based on a modified HIV-1 molecular clone which is deleted at the endogeneous protease gene and thus capable of expressing exogenous HIV-1 protease sequences. Such sequences are easily derived from clinical samples by reverse transcription and polymerase chain reaction (RT-PCR). However a person skilled in the art will realise that any other cloning method may be used for the purpose of the invention.

The HIV-1 molecular clone pNL4-3 (19, freely available at "NIH AIDS Research and Reference Reagent Program, USA, Cat. No. 114; or at MRC AIDS Reagent Project, UK, Cat No. ARP2006) was used as backbone for the construction of the protease-deleted clone. However the person skilled in the art will realise that any other HIV clone may be used for the purpose of the invention.

Briefly, a PCR-directed mutagenesis was used to obtain a deletion of the whole protease (PR) encoding sequence (including the PR/RT cleavage site) and to introduce a blunt-end restriction site to clone exogenous protease sequences. The blunt-end site results in the direct cloning of amplified exogeneous sequences, with no further processing needed. However those skilled in the art will realise that any cloning method can be utilised and is within the scope of the invention. The clone, once reconstituted by inserting an exogenous HIV-1 protease sequence, maintains a replication competence after transfection. In order to develop a non-hazardous assay, the clone was further modified by deletion of the C3-C9 region of gp120 and, in a preferred embodiment part of the RT coding sequence, thus abrogating its replication competence. The gag polyprotein expression and processing functions remained unaltered after transfection.

In order to validate the assay, the authors used the replicative form (only with the pro deletion) to evaluate the 50% inhibitory concentration (IC₅₀) of single clonal sequences to Pis. The non-replicative clone allows a wider use of this strategy, since the release of extra-cellular HIV-1 p24 antigen (p24 Ag) may be measured in the presence or in the absence of protease inhibitors, with no infectious virus produced.

It is therefore the object of the invention to establish a method to evaluate the sensitivity of an HIV variant from a biological sample to at least one drug able to inhibit the HIV protease comprising the steps of:
a) amplifying a reverse transcribed nucleic acid encoding the HIV protease from the biological sample, to obtain an amplified HIV protease coding sequence;
b) cloning the amplified HIV protease coding sequence into a unique restriction site of a modified HIV molecular clone, wherein said modified HIV molecular clone is modified at least by deleting the protease coding sequence and by replacing it with the unique restriction site, to obtain a recombinant HIV molecular clone comprising and expressing the HIV protease coding sequence;
c) introducing the recombinant HIV molecular clone comprising and expressing the HIV protease coding sequence into cultured cells, to obtain recombinant HIV transfected cells;
d) measuring the level of HIV p24 antigen in the cell supernatant of the recombinant HIV transfected cells both in the presence and in the absence of the drug able to inhibit the HIV protease.

In a preferred embodiment of the invention the method further comprises the following steps:
e) calculating the ratio R between the values of p24 antigen obtained both in the presence and in the absence of the drug able to inhibit the HIV protease;
f) normalising the ratio R to the ratio R obtained from a HIV reference sensitive protease clone.

Preferably the amplification is performed through a Reverse Transcription Polymerase Chain Reaction (RT-PCR), by use of a proof-reading thermostable DNA polymerase.

Preferably the unique restriction site is a blunt-end restriction site, more preferably is the SmaI site.

Preferably the modified HIV molecular clone is also deleted at the *env* coding sequence. More preferably the modified HIV molecular clone is also deleted at the reverse transcriptase (RT) coding sequence.

Preferably the measuring of the HIV p24 antigen is performed through an immunoenzymatic assay. The expert in the field will realise that any method suitable to detect HIV p24 antigen can be utilised. Commonly used methods relate to the quantitative detection of p24 by colorimetric immunoassays (ELISA).

Any drug able to inhibit the HIV protease can be utilised. Preferably such HIV protease inhibitors comprise indinavir, ritonavir, saquinavir, amprenavir, nelfinavir, tipranavir.

The method of the invention is suitable for any HIV virus; preferably HIV is HIV-1 or HIV-2.

It is comprised in the scope of the invention a diagnostic kit to work with the disclosed method at least comprising in sufficient amounts:
a) oligonucleotides able to prime the amplification reaction of the HIV protease coding sequence;
b) the modified HIV molecular clone of the invention, being pre-digested with the unique restriction site;
c) sensitive protease reference and positive control amplified product.

In a preferred embodiment the kit further comprises SmaI restriction endonuclease, T4 DNA ligase and ligation buffer containing ATP.

### Description of drawings

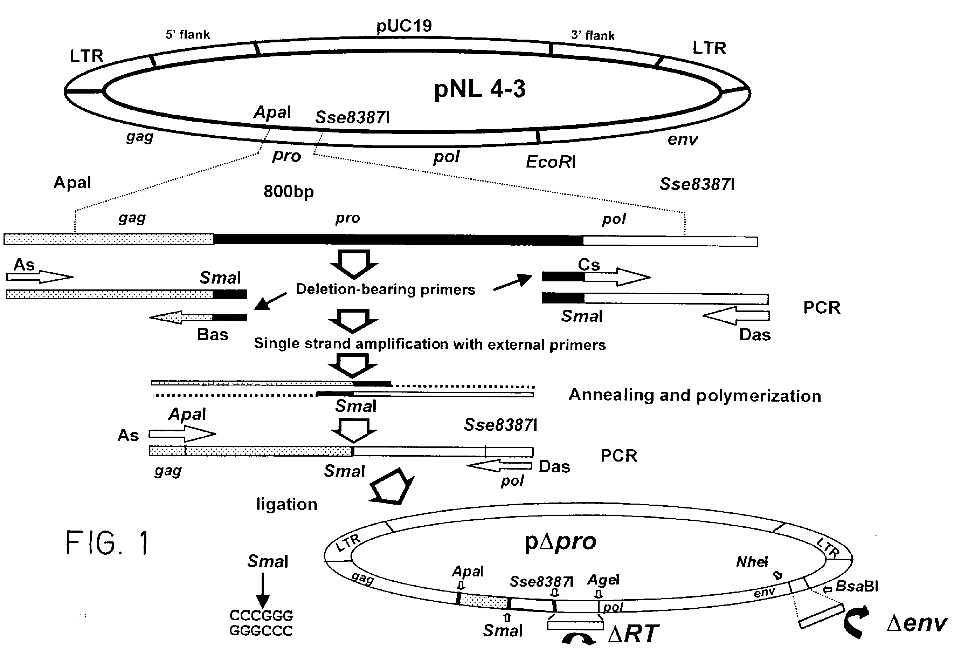

Figure 1: Development of p*Δpro*, p*ΔproΔenv* and p*ΔproΔRTΔenv* molecular clones. Two sequences of the HIV-1 *gag-pol* region were amplified using 4 primers: the external primers encompassed the *ApaI* and *Sse*83871 restriction sites. The inner primers were designated to be one the reverse complement of the other and to contain the original viral sequence in the 3' half, a blunt-end *Sma*I site in the middle, and again the viral sequence across the protease gene at the 5' end. The *Sma*I site was introduced by minimally modifying the nucleotide sequence and leaving intact the amino acid sequence. Subsequently, the antisense primer B_AS was used together with the sense prime A_S, while the sense primer C_S was coupled with the antisense primer D_AS (Table 2). Under these conditions, two separate, partially overlapping PCR products incorporated the deletion of the protease-encoding sequence and of the PR/RT cleavage site and introduced a novel *Sma*I in frame site at the splice junction. Both amplified products were used as templates in two different single-primer amplification reactions, each with its specific external primer, in order to obtain single-strand amplified products. These products were added to a primerless T7 DNA polymerase polymerization reaction, and an aliquot of this reaction was added to a new PCR reaction with primers A and D for amplification. The amplified product was restricted with *Apa*I and Sse83871 and ligated into the correspondingly cut pNL4-3 vector. Positive clones were raised and sequenced bidirectionally. One of them was grown for plasmid purification. This resulting molecular clone bears the deletion of the PR gene, the introduction of a novel, unique *Sma*I restriction site across the splice site, and no other modification within the original molecular clone sequence, thus being replication-competent after the introduction of the HIV-1 protease gene. The p*Δpro* molecular clone was modified by deleting a *Nhe*I-B*sa*BI fragment encompassing the C3-C9 region of gp120, thus producing the pΔ*pro*Δ*env* molecular clone. This clone was further modified by deleting a *Sse83871-AgeI* fragment in the RT coding sequence. These frameshift deletions abrogated the replication competence of the molecular clone, named p*ΔproΔRTΔenv.*

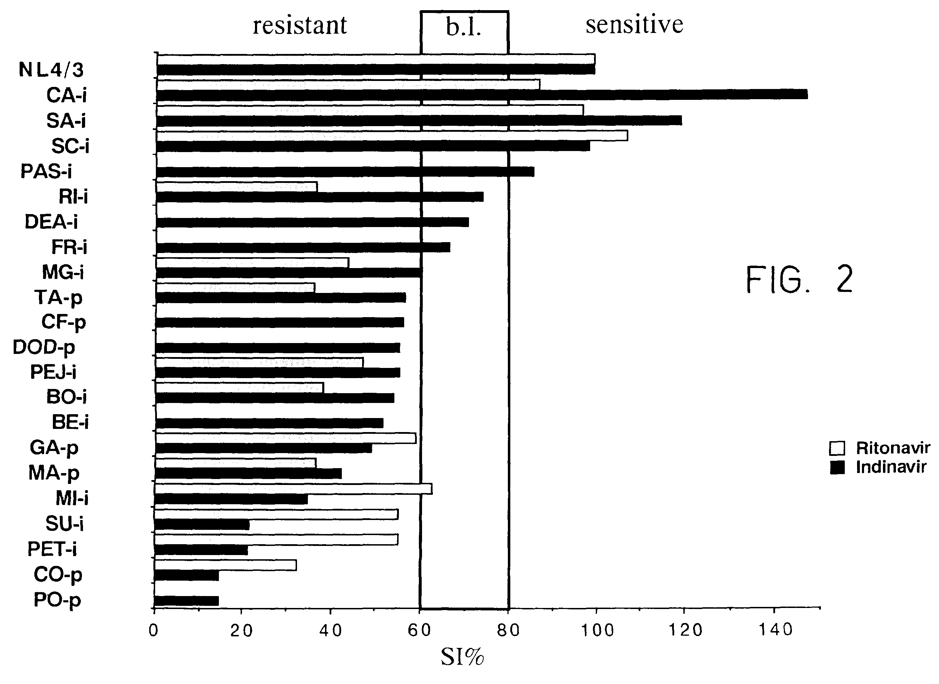

Figure 2: Percent Sensitivity Index (SI%) to Indinavir (IDV; black bars) and Ritonavir (RTV; gray bars). The amplified HIV-1 PR-encoding sequences were directly ligated into the *Sma*I predigested p*ΔproΔRTΔenv* and the ligation reaction was directly used to transform competent cells (INFO f'; Invitrogen, Groninghen, The Netherlands) by the heat shock technique. After transformation, some bacterial colonies were cultured in a new plate and new single colonies were screened by PCR with primers PR_ AS and A_S (Table 2). Positive colonies were grown in 5 mL LB medium, the plasmids extracted, and HeLa cells grown to 50% confluence on an 8-well plate (Costar), transfected by lipofectin (Bethesda Research Laboratories) in triplicate using p*ΔproΔRTΔenv* (0.5 µg) containing the exogenous HIV-1 PR sequences. One well of each recombinant was incubated in normal RPMI 1640 medium supplemented with 10% FCS, while two wells were incubated with the same medium containing IDV and RTV (both at the concentration of 0.1 µM), respectively. HIV-1 p24Ag was measured in supernatants 48 hours after transfection of the recombinant clones in the absence or in the presence of IDV and RTV. The degree of inhibition of viral expression was documented by the ratio between the levels of p24Ag detected in the presence and in the absence of the anti-viral compound (R = p24Ag^{drug}/p24Ag^{medium}). The percent Sensitivity Index (SI% = 100 x R^{reference}/R^{sample}) was introduced to normalise differences due to cell related variables among different experiments. Proteases displaying SI% values greater than 80% were considered fully sensitive; partially sensitive (intermediate phenotype) between 80 and 60%; and resistant when lower than 60%.

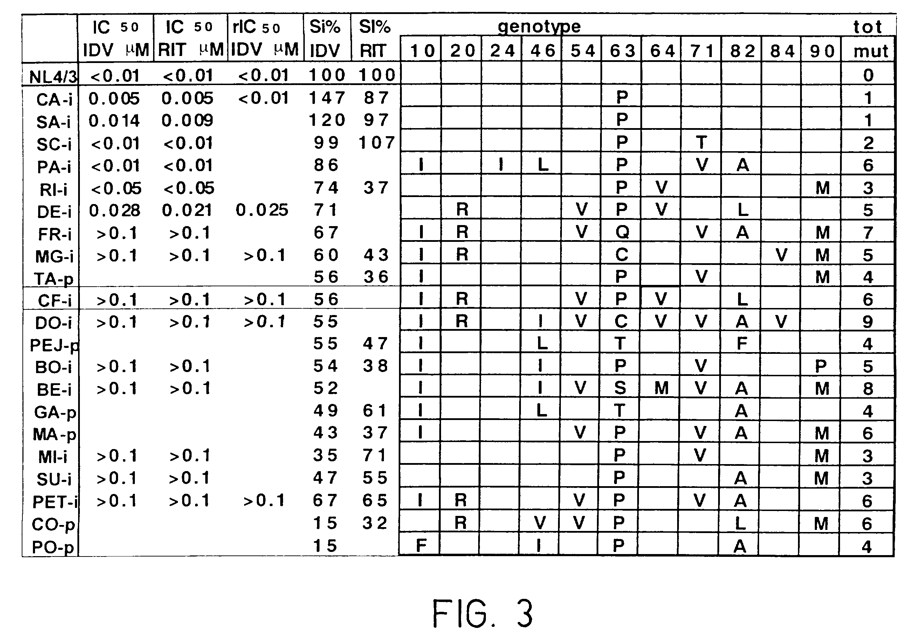

Figure 3 shows the SI% values obtained for each HIV-1 patient isolate (identified by -i) or for cell-free virus in plasma (-p) and a comparison of with the IC50 values of the original biological isolate and of the respective replicative recombinant clone (rIC50). Capital letters in the "genotype section" refer to amino acid residues relevant to the onset of resistance.

The invention will be illustrated by the following Examples.

### Example 1 - METHODS

### 1. Patients and samples

Samples from 21 HIV-1-infected subjects were used in this study (Table 1).

**Table 1**

| HIV-1 INFECTED PATIENTS FAILING TO RESPOND TO ANTI-RETROVIRAL TREATMENT | | | |
|---|---|---|---|
| Patients ID | CD4 cells/µl | HIV-1 plasma viremia (HIV-1 RNA molecules/ml) | Treatment (duration; months) |
| CA | 617 | 26,300 | No treatment |
| SC | 666 | 287,000 | No treatment |
| SA | 193 | 67,740 | 3TC,d4T,SAQ (11) |
| PA | 609 | 487,200 | 3TC,d4T,IDV (9) |
| RI | 577 | 210,000 | 3TC,d4T,SAQ (10) |
| DE | 210 | 298,000 | AZT,ddC,RIT,SAQ (6) |
| FR | 370 | 19,600 | 3TC,d4T,IDV (18) |
| MG | 20 | 77,500 | AZT,ddC,RIT (6) |
| TA | 73 | 85,000 | 3TC,d4T,IDV (12) |
| CF | 28 | 300,000 | AZT,ddI,RIT,SAQ (20) |
| DO | 407 | 380,000 | 3TC,d4T,SAQ,IDV (28) |
| PEJ | 289 | 200,000 | 3TC,d4T,IDV (14) |
| BO | 508 | 234,500 | AZT,d4T,IDV (6) |
| BE | 52 | 10,240 | AZT,ddI,IDV (18) |
| GA | 323 | 69,000 | 3TC,d4T,IDV (16) |
| MA | 364 | 90,000 | 3TC,d4T,RIT,SAQ (15) |
| MI | 175 | 450,000 | AZT,3TC,SAQ (9) |
| SU | 224 | 184,000 | 3TC,d4T,IDV (16) |
| PET | 410 | 12,000 | 3TC,d4T,IDV (20) |
| CO | 350 | 250,000 | AZT,3TC,RIT (17) |
| PO | 126 | 42,350 | AZT,d4T,IDV (20) |

Two subjects never received anti-virals, and the other 19 failed to respond to an anti-HIV-1 combination therapy, as determined by a substantial increase of HIV-1 plasma viremia. 15 out of the 19 treated subjects received combinations of 2 nucleoside analogs and 1 protease inhibitor (PI, either indinavir [IDV], or ritonavir [RTV], or saquinavir [SQV]) for 6-28 months; the remaining 4 subjects (Table 1; subjects DE, CF, DO, MA) were treated with 2 PI together with the RT inhibitors.

A viral isolate was raised from 13 out of the 19 treated subjects (from peripheral blood mononuclear cells; PBMCs) in order to establish the drug sensitivity phenotype by conventional IC₅₀ evaluation. These isolates were subjected to parallel phenotype testing using the recombinant assay (see below). To evaluate the capability of the recombinant assay of analyzing directly samples of clinical origin, plasma samples from 6 patients under combination therapy with anti-HIV-1 compounds were also included in the study.

### 2. Virus isolation and drug susceptibility assay of viral isolates

PBMCs were obtained by Fycoll (Pharmacia, Uppsala, Sweden) density gradient centrifugation of heparinized blood, and stimulated with phytohemoagglutinin (PHA-P 2µg/ml; Difco Laboratories, Detroit, MI). Uninfected PHA-P-stimulated PBMCs (5x10⁶ cells) were suspended in a final 5ml volume of R-III medium (RPMI 1640 medium supplemented with 20% heat-inactivated fetal calf serum (FCS; Hyclone, Logan, UT), 50U/ml penicillin, 50µg/ml streptomycin, 2mM L-glutamine, and 10U/ml interleukin-2 (rHuLL-2; Biosource International, Camarillo, CA) polybrene (1µg/ml; Sigma Chemical, St. Louis, MO) in 25 cm² vented tissue culture flasks (Costar, Cambridge, MA). Uninfected PBMCs were cocultured with an equal number of PBMCs from patients at a concentration of 2x10⁶ cells/ml. Cell-free supernatant fluids were harvested twice a week for HIV-1 p24Ag testing (NEN Research Products, Boston, MA), and cell-free supernatant containing breakthrough viruses were assayed for syncytium-inducing (SI) capacity using the AIDS Clinical Trials Group (ACTG) protocol for standardized detection of SI isolates (29). Viral titration was performed in PBMCs and the viral titre, measured as 50% tissue culture infectious dose (TCID₅₀)/ml, was calculated by the method of Reed and Muench.

IDV (Merck Research Laboratories, West Point, PA) and RTV (Abbott Laboratories, Abbott Park, IL) were dissolved in sterile DMSO and stored in aliquots at a concentration of 1mM at -20°C until use. Susceptibility to IDV and RTV was determined using a fixed amount of infectious virus (1,000 TCID₅₀). The viral input was used to infect 10⁶ PHA-P-stimulated PBMCs that were drug-free (control wells) or pretreated with IDV, or RTV in duplicate wells. After 7 days, HIV-1 p24Ag was measured in cell culture supernatants. As control, uninfected PBMCs were maintained at the highest concentration of the two compounds. Cell proliferation and viability were assessed by the trypan blue dye exclusion method. At different time points, the IC₅₀ of each viral isolate was determined by dose-effect analysis using the Systat computer software program for Macintosh (release 5.1). Clinical isolates were considered resistant to IDV and RTV for IC₅₀>0.1 µM.

### 3. PCR amplification of HIV-1 protease coding sequences

Cell-free genomic HIV-1 RNA was extracted from the cell culture supernatant of replicating viral isolates or from plasma samples. RNA purification was performed using QUIAquick (Qiagen GmbH, Hiden, Germany) according to the manufacturer's instructions. An aliquot of purified RNA was reverse transcribed in a 25µl reaction buffer containing 10U MoMuLV RT (Bethesda Research Laboratories, Gaithersburg, MD), 5U Rnase inhibitor (Promega Corp., Madison, WI), 1mM dNTPs, and the antisense primer PR-AS (Table 2) in a standard PCR buffer. The reaction was incubated for 30 minutes at 37°C; afterwards 75µl PCR buffer containing 2U thermostable proofreading polymerase (PWO; Boehringer Mannheim, Penzberg, Germany) and the sense PR-S primer were added and 40 amplification cycles were performed in order to obtain blunt-end amplified products.

### 4. Sequence analysis of the HIV-1 protease gene

Amplified HIV-1 PR coding genes were sequenced in both forward and reverse direction by fluorescence-labeled dideoxynucleotides, with an automated sequencer (Model 373A, Perkin Elmer, Norwalk, Co), following the sequencing conditions specified in the protocol for the ABI PRISM Dye Terminator Cycle Sequencing Kit and using Amply-Tap DNA polymerase FS (both from Perkin Elmer).

### 5. Statistical analysis

All the analyses were performed with the program StatView, version 4.5 (Abacus Concepts, Berkeley, CA). Comparisons between the SI% values (see below) and the number and type of mutations were performed using the Mann-Whitney and Kruskal Wallis tests. The correlation between the degree of sensitivity to PI of recombinant clones and the number of primary mutations relevant for PI resistance was studied by Spearman analysis.

### Example 2 - GENERATION OF pΔpro, pΔproΔenv AND pΔproΔRTΔenv MOLECULAR VECTORS

The molecular clone NL4-3 was employed as a backbone for the construction of the PR gene deleted clone. PCR-directed mutagenesis was used to obtain a deletion of the whole PR coding sequence (including the PR/RT cleavage site) and to introduce a unique *Sma*I blunt-end restriction site for cloning exogenous PR genes (Figure 1). Two different sequences of the HIV-1 *gag-pol* region were amplified using PCR. External primers encompassed the *Apa*I and Sse8371 restriction sites; the inner primers were designated to be one the reverse complement of the other and to contain: (i) the original viral sequence in the 3' half, (ii) a blunt-end *Sma*I site in the middle, and (iii) again the viral sequence across the protease gene at the 5' end (Table 2 and Figure 1).

**Table 2**

| Primer sequences | |
|---|---|
| *Amplification of the HIV-1 protease gene* | |
| Primer designation | Sequence 5'-3' |
| PR-AS (Seq ID N.1) | TGGCTTTAATTTTACTGGTAC |
| PR-S (Seq ID N.2) | TCAGATCACTCTTTGGCAACG |

| *Generation of modified HIV molecular clones* | |
|---|---|
| Primer designation | Sequence 5'-3' |
| A-S (Seq ID N.3) | GGGCACATAGCCAAAAATTG |
| B-AS (Seq ID N.4) | TGGGCCATCCATCCCGGGAAGCTAAAGGAT |
| C-S (Seq ID N.5) | ATCCTTTAGCTTCCCGGGATGGATGGCCCA |
| D-AS (Seq ID N.6) | CACATCCAGTACTGTTACTG |

Under these conditions, two separate but partially overlapping PCR products incorporated the in frame deletion of the PR-encoding sequence and of the PR/RT cleavage site, and introduced a novel *Sma*I site at the splice junction. All the introduced mutations were conservative in both the *gag* and the PR open reading frame (ORF). The amplification products were purified from primers and used as templates in two different single-primer amplification reactions (see legend to Figure 1), in order to obtain single-strand amplified products. These products were mixed, added to a primerless T7 DNA polymerase polymerization reaction and, finally, amplified using the external primers. The PCR product was purified, restricted with *Apa*I and *Sse*88371, and ligated into the correspondingly cut pNL4-3 vector. Positive clones were raised and sequenced bidirectionally. One of them was grown for plasmid purification. The molecular clone (designated p*Δpro*) bears the deletion of the PR gene, including the PR/RT cleavage site, the introduction of a novel, unique *Sma*I restriction site across the splice site, and no other modification within the original molecular clone sequence, thus being replication-competent after the introduction of the HIV-1 PR-encoding ORF.

To obtain a non-replicative molecular clone, the p*Δpro* molecular clone was modified by creating a *Nhe*I-*Bsa*BI frameshift deletion encompassing the C3-C9 region of gp120 (p*ΔproΔenv*) and further a Sse83871-AgeI deletion in the reverse transcriptase coding sequence (p*ΔproΔRTΔenv*) (Figure 1). The prototype clone p*Δpro,* maintains replication competence after transfection, once reconstituted by the insertion of an exogenous HIV-1 PR-encoding sequence; whereas the p*ΔproΔenv* and p*ΔproΔRTΔenv* molecular clones are unable to infect cells, but maintain *gag* polyprotein expression and processing functions after transfection.

### Example 3 - SINGLE-STEP EVALUATION OF THE HIV-1 PHENOTYPE TO PI AND OPTIMISATION OF THE QUANTITATIVE STRATEGY

The HIV-1 PR coding sequences amplified from the 13 culture supernatants or directly from the 6 plasma samples were cloned into the p*ΔproΔRTΔenv* nonreplicative backbone, and the resulting plasmids were transfected into semiconfluent HeLa cells. After 48 hours of incubation the supernatants were assayed for HIV-1 p24Ag concentration. The degree of inhibition of viral expression by IDV and RTV was expressed as the ratio between the HIV-1 p24 concentrations detected in the presence and in the absence of the inhibitory compound (R^{sample} = p24Ag^{drug}/p24Ag^{medium}) .

The production of HIV-1 p24Ag by the reference clone reconstructed with the NL4-3 protease was strongly inhibited in the presence of IDV and RTV, compared with production in PI-free medium, whereas in the samples obtained from patients (either from viral isolates or directly amplified from plasma) the degree of inhibition was highly variable. In the case of the NL4-3-derived sequence, the value of R varied in different experiments depending on transfection efficiency and on the conditions of transfected cells, ranging from 0.2 to 0.4, but was remarkably reproducible in different repetitions of the same experiment (CV invariably lower than 10%). For this reason, the results obtained using the reference clone and the clones from patients' samples were compared by calculating a percent Sensitivity Index (SI%). This index was determined by normalising the R value of each clinical sample (R^{sample}) to that of the NL4-3 reference strain (R^{reference}) evaluated in parallel experiment, according to the function SI% = 100 x R^{reference}/R^{sample}.

Figure 2 shows the results (SI%) obtained in viral isolates and plasma samples in the presence of IDV and RTV. The histograms document a substantially similar sensitivity to RTV and IDV for each sample, with few exceptions: sample RI-i, obtained from a patient treated with SQV, tested with SQV, tested resistant to RTV and intermediate to IDV; sample MG-i, from a patient under therapy with RTV, tested intermediate to IDV and resistant to RTV; sample MI-i, from a patient receiving SQV, tested intermediate to RTV and resistant to IDV). Figure 3 shows the SI% values obtained for each HIV-1 isolate (identified by -i) or for cell-free virus in plasma (-p). SI% values were compared with the IC₅₀ of viral isolates. An inverse relationship between SI% and IC₅₀ was observed, and SI% intervals could be established for a preliminary definition of sensitive (>80%) or resistant (<60%); these values include an intermediate phenotype. Finally, a subset of the recombinant clones (replicative forms, using the replicative clone p*Δpro*) was grown and challenged with IDV in an IC₅₀ assay (recombinant IC₅₀; rIC₅₀) . The results of these experiments overlapped with those of the corresponding HIV-1 isolates (Figure 3). These data confirm that the recombinant clones evaluated in this study reflect the phenotype to PI of the parental viruses.

### Example 4 - HIV-1 GENOTYPE AND QUANTITATIVE RECOMBINANT PHENOTYPE TO PL

The HIV-1 phenotypes to PI revealed by the quantitative recombinant strategy were compared with the genotypes of the PR-encoding ORFs cloned into the vector. Figure 3 shows the SI% and the amino acid substitutions in the residues described as relevant to the development of PI resistance (32). We analysed the relationship between SI% values and the number and type of mutations in the PR gene in both HIV-1 isolates and sequences amplified from plasma samples. Firstly, a significant inverse correlation was observed between SI% and the number of primary substitutions in the PR gene (positions 46, 82, 84 and 90) (Spearman analysis; P=0.0038 and P=0.0414 for IDV and RTV, respectively). Secondly, the PR sequences of treated patients were divided into 4 categories according to the presence of 0, 1, 2, or 3 primary mutations and the categories compared for their SI% values: significant differences could be appreciated for both IDV and RTV (Kruskal Wallis; IDV, P=0.022; RTV, P=0.0466). Thirdly, since both substitutions at positions 82 and 90 appeared to be related to a lower SI% for IDV, indicating a crucial contribution to phenotypic resistance, 3 groups of PR sequences were considered: sequences harboring (*a*) mutations at position 82 or 90 (Figure 3; 13 sequences), (*b*) mutations at positions 82 and 90 (5 sequences), and (*c*) no mutations at these sites (3 sequences). The comparison of the SI% values of the 3 groups yielded a significant difference (Kruskal Wallis; P=0.0105). Furthermore, while no different was evidenced when comparing group *a* vs. *b* (Mann-Whitney), significant results were obtained comparing *a* vs. *c* and *b* vs. *c* (P=0.0086 and P=0.0253, respectively). Together, these results highlight the reliability of the single-step, quantitative evaluation of SI%.

### Example 5 - DIAGNOSTIC KITS

A diagnostic kit comprises the following reagents:
a) oligonucleotides able to primer the amplification reaction of the HIV-1 and/or HIV-2 protease coding sequences;
b) SmaI pre-digested p*ΔproΔRTΔenv,* or alternatively SmaI pre-digested p*ΔproΔenv;*
c) sensitive protease reference and positive control amplified product.
   A more complete kit further comprises:
d) SmaI restriction endonuclease;
e) T4 DNA ligase;
f) ligation buffer containing ATP

A full complete kit comprises:
*a) Reagents for extracting HIV-1 and/or HIV-2 RNA from plasma:*
   lysis buffer;
   pH modifying buffer;
   RNA binding chromatography column;
   column wash buffer;
   RNA elution buffer;
   positive control sample.
*b) Reagents for amplifying the protease sequence:*
   RT buffer including dNTPs;
   PCR buffer including dNTPs;
   primers specific for HIV-1 and/or HIV-2;
   RNAse inhibitor;
   MoMLV (Moloney Murine Leukaemia Virus) reverse transcriptase;
   proofreading thermostable DNA polymerase;
   positive control and reference protease HIV-1 and/or HIV-2 RNA;
*c) Reagents for cloning:*
   SmaI predigested p*ΔproΔRTΔenv* molecular clone;
   sensitive protease reference and positive control amplified product;
   SmaI restriction endonuclease;
   T4 DNA ligase;
   ligation buffer containing ATP;
   competent bacteria.

### REFERENCES

1. Kahn, J.O., et al. N. Engl. J. Med. 327, 581-587 (1992).
2. Spruance, S.L., et al. Ann. Intern. Med. 126, 355-363 (1997).
3. Richman, D.D., et al. J. Virol. 68, 1660-1666 (1994).
4. Hammer, S.M., et al. N. Engl. J. Med. 355, 1081-1090 (1996).
5. Bechtold, C.M., et al. Antimicrob. Agents Chemother. 39, 374-379 (1995).
6. Deeks, S.G., et al. JAMA 277, 145-153 (1997).
7. Hammer, S.M., et al. N. Engl. J. Med. 337, 725-733 (1997).
8.Gulick, R.M., et al. N. Engl. J. Med. 337, 734-739 (1997).
9. Schmit, J.-C., et al. AIDS 10, 995-999 (1996).
10. Molla, A., et al. Nat. Med. 2, 760-766 (1996).
11. Condra, J.H., et al. Nature 374, 569-571 (1995).
12. Winters, M.A., et al. J. Virol. 72, 5303-5306 (1998).
13. Schmit, J.-C., & Weber, B. Intervirology 40, 304-321 (1997).
14. Hertogs K., et al. Antimicrob. Agents Chemother. 42, 269-276 (1998).
15. Medina, D.J. et al. J. Virol. Methods 71, 169-176 (1998).
16. Deeks, S.G. et al. J. Infect. Dis. 179,1375-1381 (1999).
17. Ratner, L. et al. AIDS Res. Hum. Retroviruses 7, 287-294 (1991).
18. Helsinger, A.A., and Antonucci, T., J. Virol. Methods 49, 247-255 (1994).
19. Adachi A., et al., Journ. Virol., 59, 284 (1986).

sequence

<400> 3
gggcacatag ccaaaaattg                                                    20

<210> 4
<211> 30
<212> DNA
<213> Artificial Sequence

<220>
<223> Description of Artificial Sequence: primer
      sequence

<400> 4
tgggccatcc atcccgggaa gctaaaggat                                         30

<210> 5
<211> 30
<212> DNA
<213> Artificial Sequence

<220>
<223> Description of Artificial Sequence: primer
      sequence

<400> 5
atcctttagc ttcccgggat ggatggccca                                         30

<210> 6
<211> 20
<212> DNA
<213> Artificial Sequence

<220>
<223> Description of Artificial Sequence: primer
      sequence

<400> 6
cacatccagt actgttactg                                                    20

SEQUENCE LISTING

<110> Biostrands s.r.l.

<120> Method to evaluate the HIV drug sensitivity

<130> EP

<140>
<141>

<160> 6

<170> PatentIn Ver. 2.1

<210> 1
<211> 21
<212> DNA
<213> Artificial Sequence

<220>
<223> Description of Artificial Sequence: primer
      sequence

<400> 1
tggctttaat tttactggta c                                                21

<210> 2
<211> 21
<212> DNA
<213> Artificial Sequence

<220>
<223> Description of Artificial Sequence: primer
      sequence

<400> 2
tcagatcact ctttggcaac g                                                21

<210> 3
<211> 20
<212> DNA
<213> Artificial Sequence

<220>
<223> Description of Artificial Sequence: primer

## Claims

1. Method to evaluate the sensitivity of an HIV variant from a biological sample to at least one drug able to inhibit the HIV protease comprising the steps of:
a) amplifying a reverse transcribed nucleic acid encoding the HIV protease from the biological sample, to obtain an amplified HIV protease coding sequence;
b) cloning the amplified HIV protease coding sequence into a unique restriction site of a modified HIV molecular clone, wherein said modified HIV molecular clone is modified at least by deleting the protease coding sequence and by replacing it with the unique restriction site, to obtain a recombinant HIV molecular clone comprising and expressing the HIV protease coding sequence;
c) introducing the recombinant HIV molecular clone comprising and expressing the HIV protease coding sequence into cultured cells, to obtain recombinant HIV transfected cells;
d) measuring the level of HIV p24 antigen in the cell supernatant of the recombinant HIV transfected cell culture both in the presence and in the absence of the drug able to inhibit the HIV protease.

2. Method to evaluate the sensitivity of an HIV variant from a biological sample to at least one drug able to inhibit the HIV protease according to claim 1 further comprising the following steps:
e) calculating the ratio R between the values of p24 antigen obtained both in the presence and in the absence of the drug able to inhibit the HIV protease;
f) normalising the ratio R to the ratio R obtained from a HIV reference sensitive protease clone.

3. Method to evaluate the sensitivity of an HIV variant from a biological sample to at least one drug able to inhibit the HIV protease according to claim 1 or 2 wherein the amplification is performed through a Reverse Transcription Polymerase Chain Reaction (RT-PCR), by use of a proof-reading thermostable DNA polymerase.

4. Method to evaluate the sensitivity of an HIV variant from a biological sample to at least one drug able to inhibit the HIV protease according to any of previous claims wherein the unique restriction site is a blunt-end restriction site.

5. Method to evaluate the sensitivity of an HIV variant from a biological sample to at least one drug able to inhibit the HIV protease according to claim 4 wherein the blunt-end restriction site is the SmaI site.

6. Method to evaluate the sensitivity of an HIV variant from a biological sample to at least one drug able to inhibit the HIV protease according to any of previous claims wherein the modified HIV molecular clone is also deleted at the *env* coding sequence.

7. Method to evaluate the sensitivity of an HIV variant from a biological sample to at least one drug able to inhibit the HIV protease according to any of previous claims wherein the modified HIV molecular clone is also deleted at the *RT* coding sequence.

8. Method to evaluate the sensitivity of an HIV variant from a biological sample to at least one drug able to inhibit the HIV protease according to any of previous claims wherein the measuring of the HIV p24 antigen is performed through an immunoenzymatic assay.

9. Method to evaluate the sensitivity of an HIV isolate from a biological sample to at least one drug able to inhibit the HIV protease according to any of previous claims wherein the drug able to inhibit the HIV protease is comprised in the group of: indinavir, ritonavir, saquinavir, amprenavir, nelfinavir, tipranavir.

10. Method to evaluate the sensitivity of an HIV isolate from a biological sample to at least one drug able to inhibit the HIV protease according to any of previous claims wherein HIV is HIV-1 or HIV-2.

11. Diagnostic kit to work the method according to any of previous claims at least comprising in sufficient amounts:
a) oligonucleotides able to prime the amplification reaction of the HIV protease coding sequence;
b) the modified HIV molecular clone of the invention, being pre-digested with the unique restriction site;
c) sensitive protease reference and positive control amplified product.

12. Diagnostic kit according to claim 11 further comprising in sufficient amounts: SmaI restriction endonuclease, T4 DNA ligase and ligation buffer containing ATP.
